# EUROPEAN PATENT APPLICATION

(11) **EP 1 862 535 A1**
(43) Date of publication of application: **05.12.2007**
(21) Application number: 07010620.8
(22) Date of filing: 29.05.2007
(51) Int. Cl.: C12M 3/06, C12N 5/00

(54) **Cell processing method and cell processing apparatus**

(30) Priority: 30.05.2006 JP 2006150005; 09.11.2006 JP 2006304086
(71) Applicant: SYSMEX CORPORATION, Kobe-shi, Hyogo 651-0073 (JP)
(72) Inventor: Nagaoka, Kanako, Kobe-shi Hyogo 651-0073 (JP); Ishisaka, Masaki, Kobe-shi Hyogo 651-0073 (JP)
(74) Representative: HOFFMANN EITLE

(57) **Abstract**

A cell processing method for processing and collecting cells in a cell suspension fluid with reagent comprising: line filtering a cell suspension fluid using a membrane filter (8); processing the cells ion the membrane filter with reagent; supplying a collection fluid to produce a shearing force that acts on the cells processed with reagent so as to separate the cells on the membrane filter into the collection fluid; and recovering the collection fluid that retains the cells separated from the membrane filter.

## Description

### FIELD OF THE INVENTION

The present invention relates to a cell processing method and cell processing apparatus capable of high efficiency collection, via a membrane filter, of cells in an optional fluid after reagent treatment of cells in a cell suspension fluid.

### BACKGROUND

Normally uterine cervical cell examination is used as a screening method of uterine cervix cancer. Uterine cervical cell examinations are performed by scraping the surface of the uterine cervix using cotton swab or a specialized collecting tool such as scraper, preparing a specimen by smearing the collected cells instantly on a slide glass, and microscopically examining the specimen. Alternatively, when a large quantity of specimens must be examined as in the case of regular health examinations, specimens are prepared by preserving the collected scraped cells in a fixing solution and transferring them to an examination laboratory where a smear specimen is prepared, staining the specimen with Papanicolaou stain, and obtaining pathological diagnostic information via microscopic examination.

Since smear samples must be prepared and microscopically examined for individual specimens in either case, the process is complex and time consuming, and requires a high level of diagnostic skill, which has led in recent years to attempts to automate cytologic examinations.

Flow cytometry is an example of an automation method for cytologic examinations. Flow cytometry is a method in which a plurality of information is obtained from a large quantity of cells in a short time by immunizing cells with an optional fluorescence-labeled antibody, flowing a cell-containing suspension fluid through a flow cell and irradiating the suspension fluid with laser light, and measuring the fluorescence information and scattered light information emitted by the individual cells, then performing correlative and statistical analyses using the obtained information. In cytologic examinations, the presence or absence of cancerous cells can be determined by selecting an optional disease (cancer)-dependent protein as a marker, and using flow cytometry to measure a suspension of cells treated by a fluorescence-labeled antibody that is specific for the marker protein. The cell staining process necessarily includes a reaction process between the cells and a reagent, and a cell washing process via a solution exchange.

An example of a method used to prepare measurement samples for use in flow cytometry is a centrifugation method using the difference in the specific gravities of the cells and the solution. However, the centrifugation method is not a technique that is suited to automation from the perspectives of safety and the scale of the cell processing apparatus. An alternative method to centrifugation is a filtration method that utilizes the size of the cell.

United States Patent Publication No. 6,139,757 discloses art for filtering and collecting cells by changing the pore diameter of a membrane filter by utilizing elastic deformation thereof. That is, the art disclosed in United States Patent Publication No. 6,139,757 pertains to capturing cells using a membrane filter which has a small pore diameter in a compressed condition, and thereafter increasing the pore diameter of the membrane filter in a non-compressed condition and separating the captured cells from the membrane filter by supplying liquid from the opposite direction.

The art disclosed in United States Patent Publication No. 6, 139,757 is disadvantageous, however, because a device is required to compressed and expand the membrane filter and a plurality of membrane filters are used, the process becomes complicated and the cost of the apparatus increases. Moreover, a large quantity of collection liquid is required to collect cells, and the low cell concentration in the collection liquid is problematic insofar as it is unsuited for the preparation of measurement samples used in flow cytometry.

### SUMMARY

The scope of the present invention is defined solely by the appended claims, and is not affected to any degree by the statements within this summary.

A first aspect of the present invention is a cell processing method for processing and collecting cells in a cell suspension fluid comprising the steps of:
filtering a cell suspension fluid in which cells are suspended using a membrane filter to capture the cells on the membrane filter;
processing the cells on the membrane filter with reagent;
supplying a collection fluid to the membrane filter to produce a shearing force that acts on the cells processed with reagent and transfer the cells from the membrane filter into the collection fluid; and
collecting the collection fluid in which the cells transferred from the membrane filter are suspended.

A second aspect of the present invention is a cell processing apparatus for processing cells using a membrane filter comprising:
a setting section in which a holder having a membrane filter is set;
a first dispensing section for dispensing a cell suspension fluid that contains cells to the holder set in the setting section;
a second dispensing section for dispensing a reagent to the holder set in the setting section;
a liquid supplying section for supplying a separating liquid to separate the cells from the membrane filter in the holder arranged in the setting section; and
a control section for controlling the first dispensing section to dispense the cell suspension fluid to the holder, the second dispensing section to dispense the reagent to the holder to which the cell suspension fluid has been dispensed, and the liquid supplying section to supply the separating liquid to separate the cells treated with the reagent from the membrane filter in the holder to which the reagent has been dispensed.

### BRIEF DESCRIPTION OF THE DRAWINGS

Fig. 1 is a brief perspective view of a first embodiment of the cell processing apparatus;
Fig. 2 is a brief top view of a first embodiment of the cell processing apparatus;
Fig. 3 is a brief perspective view of the collection fixture moving device;
Fig. 4a is a brief structural view of the collection fixture of the first embodiment;
Fig. 4b is a brief structural view of the collection fixture of the first embodiment;
Fig. 4c is a brief structural view of the collection fixture of the first embodiment;
Fig. 5a illustrates the pressure condition during cell recovery using the collection fixture of the first embodiment;
Fig. 5b illustrates the pressure condition during cell recovery using the collection fixture of the first embodiment;
Fig. 6 illustrates the result of cell recovery in the first embodiment;
Fig. 7 shows another example of the first embodiment;
Fig. 8 is a top plan view of the cell processing apparatus of the first embodiment;
Fig. 9 is an air circuit diagram of the holder setting section in the cell processing apparatus of Fig. 1;

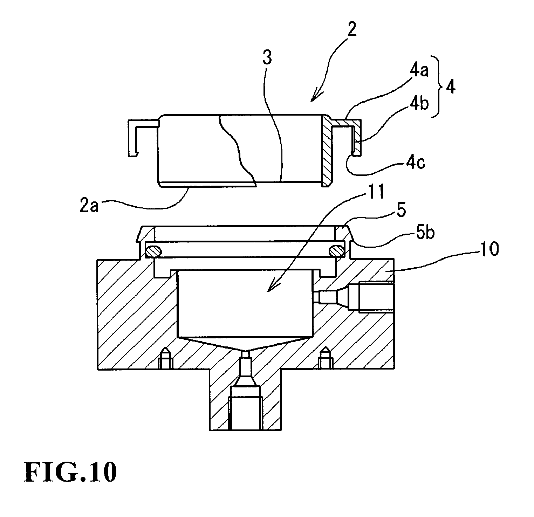

Fig. 10 is a cross section view of the holder setting section and the holder with a filter;

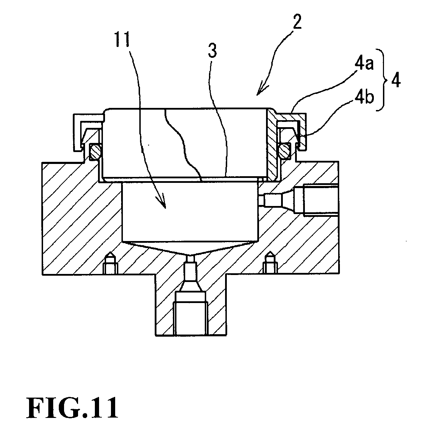

Fig. 11 is a cross section view of the holder setting section on which a holder is mounted;
Fig. 12 is a cross section view of the vicinity at the end of the holder;
Fig. 13 is a cross section view of the holder setting section and the reagent dispensing unit;
Fig. 14 shows the collection fluid supplying member of the collection fluid supplying unit viewed from below;
Fig. 15 shows the collection fluid supplying member of the collection fluid supplying unit viewed from above;
Fig. 16 is a cross section view of the washing unit;
Fig. 17 shows the units which comprise the cell processing apparatus;
Fig. 18 illustrates the control section for controlling the cell processing apparatus;
Fig. 19 is a flow chart showing an example of the cell processing performed by the cell processing apparatus of the first embodiment;
Fig. 20 is a flow chart showing an example of the cell processing performed by the cell processing apparatus of the first embodiment;
Fig. 21 is a flow chart showing an example of the cell processing performed by the cell processing apparatus of the first embodiment;
Fig. 22 is a flow chart showing an example of the cell processing performed by the cell processing apparatus of the first embodiment;
Fig. 23 is a flow chart showing an example of the cell processing performed by the cell processing apparatus of the first embodiment; and
Fig. 24 is a flow chart of the aspiration process of the first embodiment.

### DETAILED DESCRIPTION OF THE PREFERRED EMBODIMENT

### (First Embodiment)

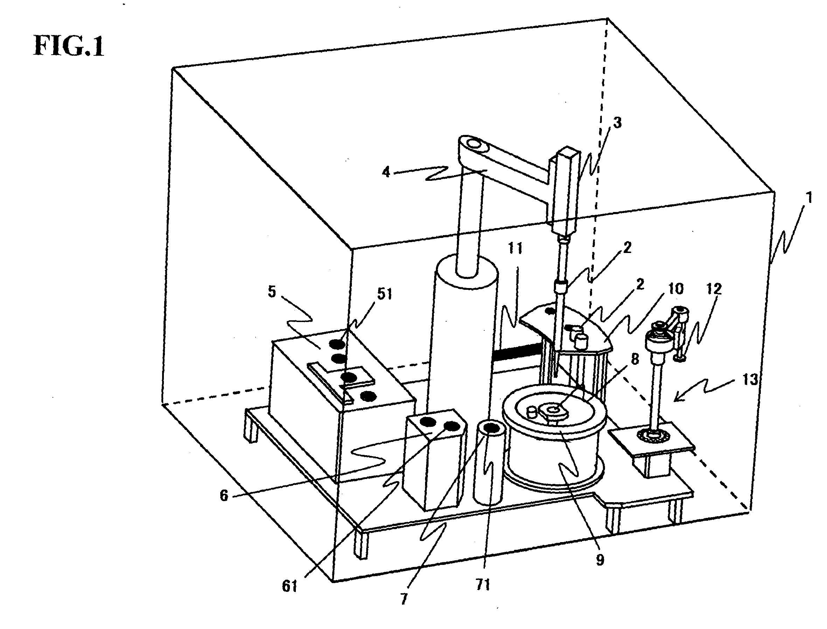

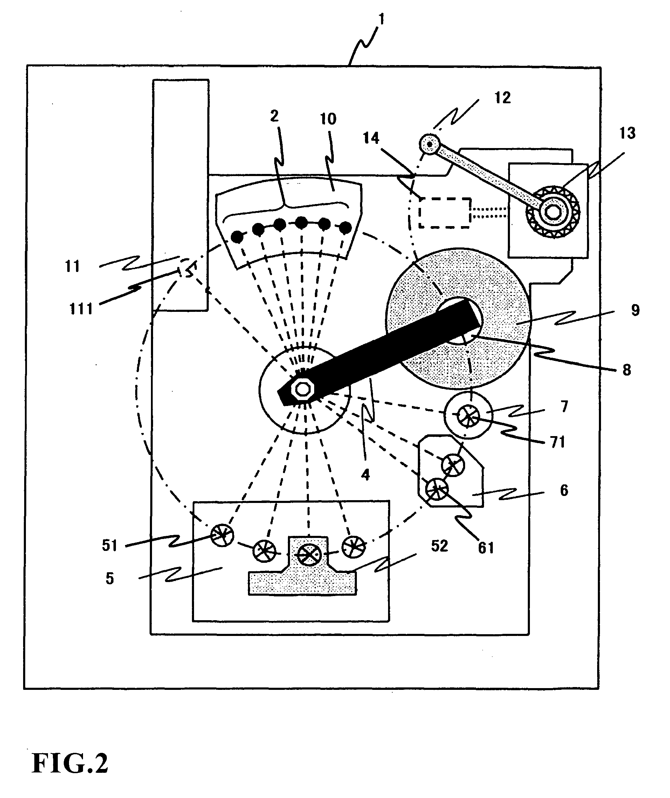

Fig. 1 is a brief perspective view of the first embodiment of the cell processing apparatus of the present invention, and Fig. 2 is a top view of same. The cell processing apparatus 1 filters cells in a cell suspension fluid using a membrane filter, performs a reagent process using a staining solution and a washing process using a washing liquid on the cells captured by the membrane filter, and subsequently recovers the cells on the membrane filter in a collection fluid.

The cell processing apparatus 1 includes a robot arm 4 for holding a pipette 3 with an installed disposable tip 2, reagent cooling box 5 for receiving the reagent required for storage, reagent room-temperature storing box 6 for receiving the reagent required for room-temperature storage, washing liquid storage box 7 for storing washing solution, reaction chamber 9 which is capable of internally deploying a membrane filter 8, disposable tip receiver 10 for supporting the replacement disposable tips, tip waste section 11 for disposing of the used disposable tips removed from the pipette 3, collection fixture moving device 13 for deploying the collection fixture 12 on the membrane filter 8 within the reaction chamber 9, and syringe pump 14 for supplying collection fluid to the collection fixture 12.

The robot arm 4 is configured so as to be rotatable in any direction about the Z-axis, and vertically movable along this axis.

The reagent cooling box 5 cools the reagent by means of a Peltier cooling device not shown in the drawing, and the top surface of which is provided with reagent supplying ports 51 for supplying reagents. Each reagent supplying port 51 is covered with a slit-type rubber cap to prevent evaporation of the reagent, and a disposable tip installed on a pipette can be inserted the slit of the rubber cap.

The reagent room-temperature storing box 6 accommodates reagent that can be stored at room temperature, and the top part of which is provided with reagent supplying ports 61. The reagent supplying ports 61 are covered with a lit-type rubber cap similar to the reagent supplying ports 51. The washing liquid storing box 7 holds washing liquid, and has washing liquid supplying ports 71, the top part of which are provided with slit-type rubber caps. The disposable tip receiver 10 holds replacement disposable tips 2. After the pipette 3 which does not have an installed pipette tip has been moved above a desired disposable tip 2 by the robot arm 4, the pipette 3 is lowered and the disposable tip 2 is installed on the pipette 3. Moreover, a used disposable tip 2 is inserted into a tip disposal port 111 of the tip waste section 11 for disposal.

As shown in Fig. 2, each reagent supplying port 51 of the reagent cooling box 5, reagent supplying ports 61 of the reagent room-temperature storing box 6, buffer solution supplying ports 71 of the buffer solution storing box 7, membrane filter 8 deployed within the reaction chamber 9, replacement disposable tips 2 of the disposable tip receiver 10, and tip disposal port 111 of the disposable tip waste section 11 are arranged on the circular track of the pipette 3 rotated by the robot arm 4.

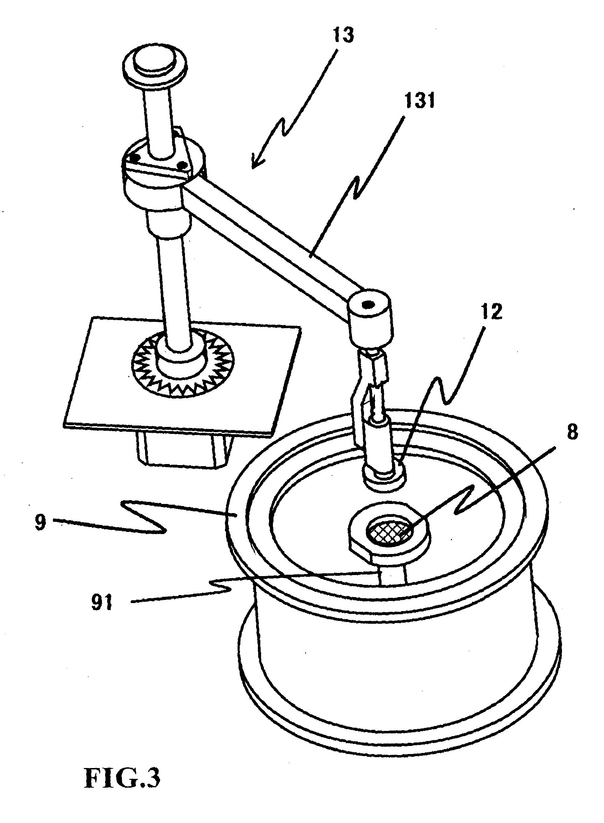

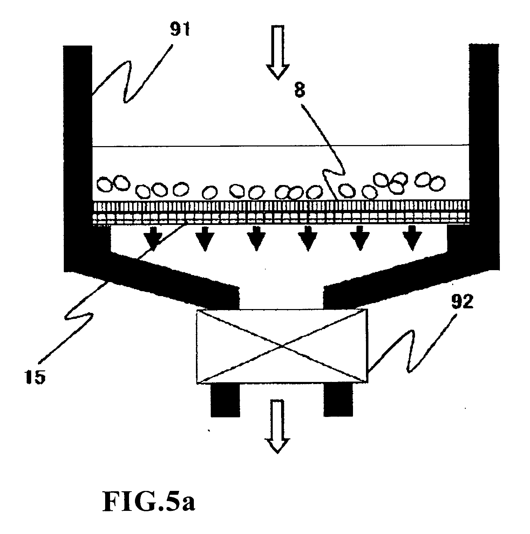

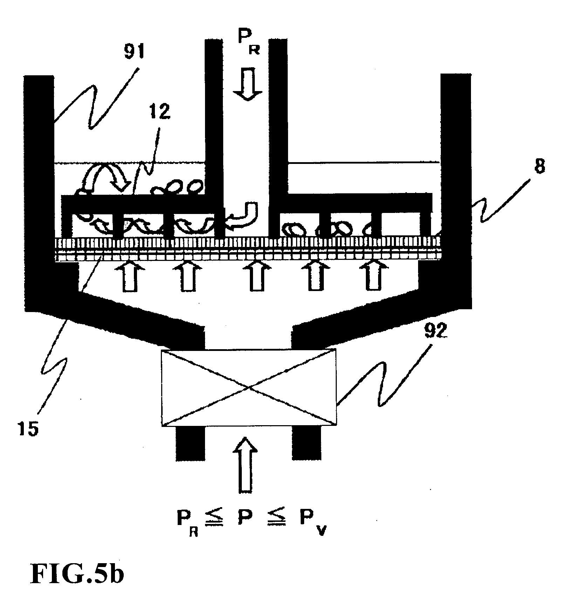

As shown in Figs. 3 and 5, the reaction chamber 9 is internally provided with a filter holding member 91 and filter supporting body 15, whereby the membrane filter 8 is held by the holding member 91, and the membrane filter 8 is preventing from flexing under the hydraulic pressure within the filter by the supporting body 15. The filter supporting body 15 is unnecessary when the membrane filter 8 is not deformed by the hydraulic pressure. The bottom part of the holding member 91 is connected to the an air pressure source which is not shown in the drawing via a valve 92 (Fig. 5), and applies pressure or suction from the back surface of the membrane filter 8. The bottom part of the holding member 91 is connected to a waste liquid receptacle, and filtered liquid, used reagent, and wash solution and the like are collected in the waste liquid receptacle. The reaction chamber 9 functions as a thermostat bath when filled with hot water supplied from a hot water supplying device not shown in the drawing, so as to be capable of regulating the temperature cells are subjected to reagent processing. The membrane filter 8 is removably held by the holding member 91, and is replaced after processing each single sample of cell suspension fluid.

The collection fixture moving device 13 is rotatable with the Z-axis as the rotational axis, and is provided with a vertically movable arm 131, and collection fixture 12 detachably held by the arm 131. Furthermore, the collection fixture moving device 13 is connected to the syringe pump 14 via a tube. Collection fluid supplied from the syringe pump 14 can be supplied to the collection fixture 12 through a tube provided within the collection fixture moving device 13.

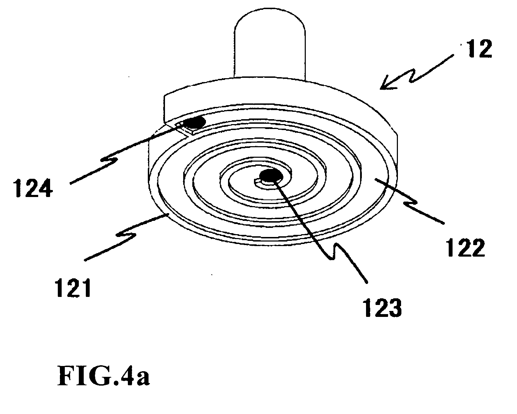

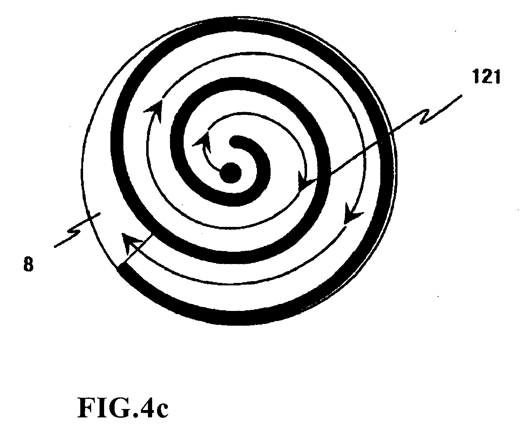

The collection fixture 12 is described with reference to Figs. 4a through 4c. The collection fixture 12 is provided with a spiral spacer 121 provided on the bottom surface to regulate the spacing relative to the membrane filter 8 when disposed on the membrane filter 8 and form a flow path 122 for the collection fluid on the membrane filter 8, collection fluid supplying port 123 for supplying collection fluid to the flow path 122, collection fluid discharge port 124 for discharging collection fluid that contains cells separated from the membrane filter 8, and reservoir 125 for storing the collection fluid discharged from the collection fluid discharge port 124. The collection fixture 12 changes the flow of the collection fluid supplied through the collection fluid supplying port 123 to the membrane filter 8 in a perpendicular direction to a flow parallel to the membrane filter 8, and produces a shear flow relative to the cells on the membrane filter 8. Fig. 4c shows the flow of the collection fluid viewed from above the collection fixture. Since the collection fluid supplied through the collection fluid supplying port 123 exerts a shearing force on the cells on the membrane filter 8 along the spiral flow path regulated by the spacer 121, cells can be effectively separated from the membrane filter 8 via the action of the uniform shear force on the cells. This shear force can be adjusted by the flow volume per unit time of the supplied collection fluid, height of the spacer, and the width of the flow path formed by the spacer 121, and thus can be suitably set in accordance with the amount and condition of the cells to be processed.

The cell processing method using the previously described cell processing apparatus 1 is described below. In the method of the present embodiment, the cells in the cell suspension fluid are cells collected from a patient, and information aiding diagnosis is obtained through examination of these cells. In diagnosing cervical cancer, for example, a cell suspension fluid is used in which cells originating in the uterus are suspended in a solvent.

A cell suspension fluid is first supplied to the membrane filter 8 held by the holding member 91 and supported by the supporting body 15 and deployed within the reaction chamber 9. As shown in Fig. 5, the cell suspension fluid supplied to the membrane filter 8 is suctioned and filtered by a negative pressure exerted from the back surface side of the membrane filter 8. The suction filtration is performed so as to have the solvent in the cell suspension to remain together with the cells on the membrane filter 8. The presence of both the solvent and cells on the membrane filter 8 prevents solidification of the cells on the membrane filter 8 and improves the efficiency of cell collection which is described later. When the cells must be incubated in accordance with the type of reagent used, hot water of a predetermined temperature is supplied beforehand to the reaction chamber 9.

After the pipette 3 has been moved above the disposable tip receiver 10 by the robot arm 4, the pipette 3 is lowered in a vertical direction and a disposable tip 2 is installed on the leading end of the pipette 3. After the pipette 3 with the installed disposable tip 2 is raised in a vertical direction, the pipette 3 is moved above the reagent supplying port 51 of the reagent cooling box 5, the pipette 3 is subsequently lowered and inserted into the reagent supplying port 51, and aspirates a predetermined quantity of reagent. After the pipette 3 which has aspirated reagent through the disposable tip 2 has been raised in a vertical direction, the pipette 3 is lowered toward the membrane filter 8.

Then, the pipette 3 discharges the reagent onto the membrane filter 8. At this time, the valve 92 provided in the bottom part of the holding member 91 is closed to allow sufficient reaction between the cells and the reagent. After the reagent is completed, the valve 92 is opened and the reagent is removed by suctioning.

After the pipette 3 has been moved above the tip disposal port 111 of the tip waste section 11 by the robot arm 4, the pipette 3 is lowered and the disposable tip 2 is inserted into the tip disposal port 111 and removed. A disposable tip 2 is again installed on the pipette 3 in the disposable tip receiver 10, and in the washing liquid storing box 7 the washing fluid is suctioned and supplied to the cells previously processed with reagent on the membrane filter 8, then the washing fluid is removed by suctioning from the back side of the membrane filter 8. The cells are processed with a different reagent and washed as necessary. The suction removal of washing fluid and suction removal of reagent after the completed reaction are performed so as to have some solvent remaining on the membrane filter 8 to prevent solidification of the cells on the membrane filter 8.

After washing, the reagent-processed cells are collected using the collection fixture 12. The collection fixture 12 is mounted above the reagent-processed cells by the collection fixture moving device 13. When the collection fixture 12 is disposed above the membrane filter 8, the collection fluid is supplied from the syringe pump 14 to the membrane filter 8 from the collection fluid supplying port 123 of the collection fixture 12. At this time, the air exerts a back pressure P from the back side of the membrane filter 8, to prevent leakage through the membrane filter 8 due to the feed pressure pR of the collection fluid supplied from the collection fixture 12, as shown in Fig. 5b. The back pressure pR is set so as to be a lower value than the bubble point pVP of the membrane filter 8 to prevent air passing through the membrane filter 8. When the collection fluid is supplied from the collection fluid supplying port 123 onto the membrane filter 8 in this condition, a shear flow is formed that effectively prevents leakage. The collection fluid separates the cells from the membrane filter 8 by the shear force created when flowing through the flow path regulated by the spiral spacer 121 provided on the bottom surface of the collection fixture 12, and is finally collected in the reservoir 125provided on the top surface of the collection fixture 12.

The collection of cells on the membrane filter 8 by the shear flow produced using the collection fixture 12 has been experimentally verified. A circular membrane (pore diameter: 1 µm; effective membrane diameter: 10 mm) of hydrophilic polytetrafluoroethylene (PTFE) can be used as the membrane filter 8 held in the membrane filter holding member 91 and supported by the membrane filter supporting body 15.

A cell suspension fluid sample was prepared from the uterine cervical scraping specimen, and contained approximately 1×10⁵ cells in 1 ml fixing solution (PreservCyto; Cytyc, Inc.). The cells were pre stained with fluorescent dye (Hoechst 33342) for microscopic examination of the cells remaining on each membrane filter 8 after testing.

One milliliter of the cell suspension sample was dripped onto the membrane filter 8 and suctioned at 2 ml/min by applying a negative pressure at the back surface of the membrane, and the valve 91 was closed so that 50 µl solvent of the cell suspension fluid remained on the circular membrane. Then, 1 ml of phosphate-buffered solution was dripped onto the same membrane as a cell washing solution and suction filtered at 2 ml/min, and the valve 92 was closed so that 50 µl solvent remained on the membrane. The washing solution was similarly dripped and suctioned a total of eight times.

Next, the collection fixture 12 was deployed on the membrane so that the bottom surface of the spacer 121 made contact with the membrane. The collection fixture had a circular external diameter that matches the effective diameter of the membrane filter being used, and the height of the spacer 121 from the bottom surface was 0.2 mm with a gap (flow path width) from the spacer of 2 mm. 200 µl of collection fluid (Ret sheath; Sysmex, Corporation) was supplied from the collection fluid port 123 to the membrane at flow volume of 4 ml/min. At this time, air pressure was increased from the back side of the membrane to prevent the collection fluid from passing through the membrane. The collection fluid was transformed to a flow parallel to the membrane according to this configuration, and flowed from the center to the margin along the spiral flow path formed by the spacer 121. The cells captured on the membrane were separated therefrom and released in the collection fluid by the shear flow, which was nearly uniform over the entire membrane surface, produced by the collection fluid flowing in the flow path. The collection fluid was collected in the reservoir 125 provided on the top surface of the collection fixture 12.

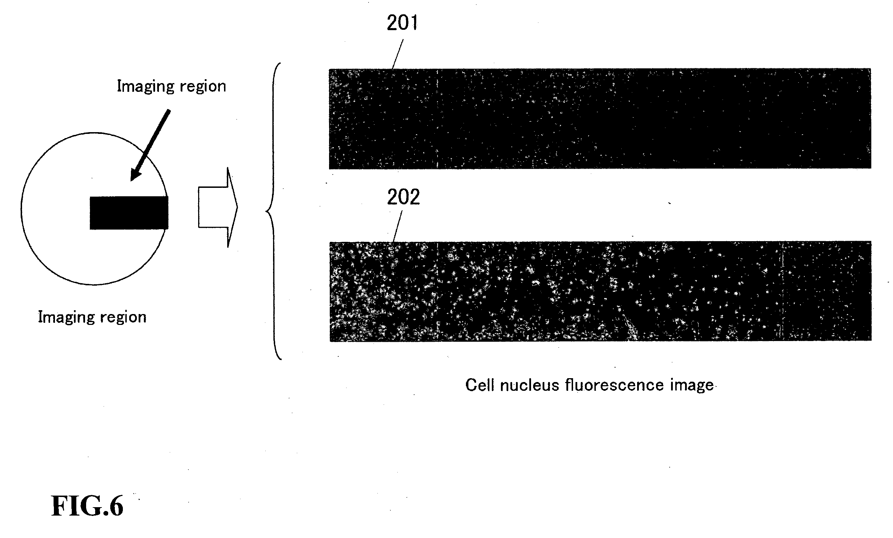

The collection fluid volume was approximately 170 µl. The number of cells collected in the collection fluid relative to the number of cells supplied to the membrane filter was 85%. After the collection fluid was recovered, the cells remaining on the membrane were detected for each membrane by fluorescence microscopy. The results are shown in image 201 of Fig. 6. Cells in the drawing are indicated by bright points. As can be understood from image 201 of Fig. 6, residual cells are nearly absent across the entire surface of the membrane.

For comparison, image 202 in Fig. 6 is a fluorescence image of a membrane which only filtered the cell suspension fluid. By comparing images 201 and 202, it is clear that the method of the present embodiment is capable of recovering substantially all cells in a minute amount of collection fluid, that is 200 µl from the membrane, thus confirming that collection fluid can be supplied so as to produce an effective uniform shear force on the cells on the membrane filter across the entire surface of the membrane filter. There was no observed morphological change produced in the cells in the obtained collection fluid caused filtration and collection.

Furthermore, the fact that the volume of the collection path was 20 µl and the fact that 170 µl of collection fluid was used confirms that supplying air from the back side of the membrane filter at a pressure below the bubble point of the membrane filter but greater than the hydraulic pressure of the collection fluid prevented the collection fluid from passing through the membrane filter and efficiently formed an effective shear flow.

Although a circular membrane made of hydrophilic tetrafluoroethylene was used as the membrane filter 8 in the present embodiment, the present invention is not limited to this material, and the material used is not restricted insofar as the material has superior durability and protein does not easily absorbed by the material. The membrane filter is preferably hydrophilic from the perspective of preventing protein absorption. Moreover, the shape of the membrane filter is not specifically limited, for example, a polygonal shape rather than a circular shape may be used.

The hydrophilic membrane filter may be formed of hydrophilic polymer, or the surface of the membrane filter may be coated with a hydrophilic polymer material. Although the hydrophilic polymer material is not specifically limited, examples of desirable materials include hydrophilic polytetrafloroethylene, hydrophilic nylon, hydrophilic polyvinylidene fluoride, hydrophilic polycarbonate, hydrophilic polypropylene, hydrophilic polysulfone, and hydrophilic polyether sulfone.

The pore diameter of the membrane filter is not specifically limited insofar as the objective cells in the cell suspension fluid can pass through the pores. The selection of a suitable pore diameter is dependent on the size of the object cell.

Although the total percentage of void created by the membrane filter and filter supporting body is not specifically limited, this void is desirably adjusted in accordance with the size and number of cells to be processed since when there is an excessive number of cells per sample of cell suspension fluid, there is an increase in the load on the cells by the membrane filter which prevents efficient filtration.

Although suctioning (addition of negative pressure) is performed from the back surface of the membrane filter (the opposite side relative to the surface of cell existence) when cells in the cell suspension fluid are filtered by the membrane filter 8 of the present embodiment, the hydraulic pressure of the cell suspension fluid may also be used alone under atmospheric pressure.

It is desirable that suctioning is terminated while observing the fluid volume on the membrane filter in order to leave both cells and solvent of the cell suspension fluid on the membrane filter during filtration. For example, the fluid volume on the membrane filter can be monitored by observing the suctioned volume, observing the liquid level of the cell suspension fluid via an optical sensor, or observing the change in pressure on the back side of the membrane filter via a pressure sensor.

In the present embodiment, the collection fluid is supplied in a flow perpendicular to the membrane filter and transformed to a parallel flow to the membrane filter by the collection fixture 12 in order to supply the collection fluid to produce a shear force that acts on the cells on the membrane filter 8, however, the present invention is not limited to this method. The collection fluid may also be supplied in a parallel flow to the membrane filter 8 from the start, or may be supplied in a non-parallel flow other than perpendicular flow to the membrane filter and transformed into a parallel flow relative to the membrane filter 8. The collection fluid is not specifically limited insofar as the fluid preserves the cells without morphological damage. It is desirable that, when deployed on the membrane filter 8, the collection fixture 12 has a shape that forms one or more collection fluid flow paths with a supplying port at one end and a discharge port at the other end, such that the bottom surface is the cell existence surface of the membrane filter 8 in the region of cell existence on the membrane filter 8.

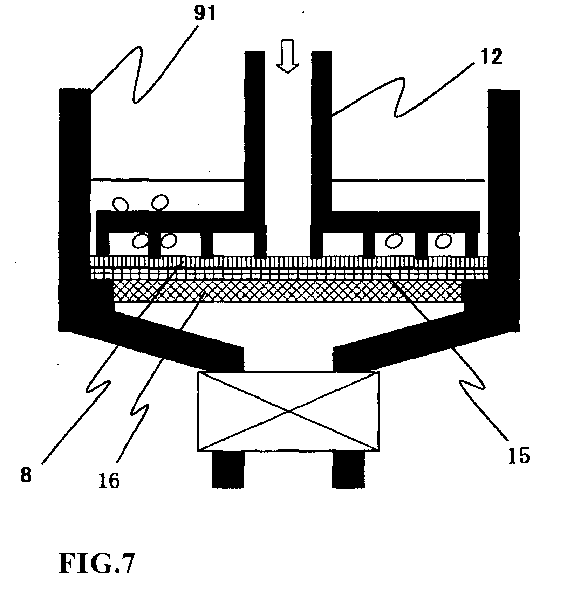

Although leakage of the flow is prevented when collection fluid is supplied by adding backpressure using air from the back surface of the membrane filter 8 in the present embodiment, a leak preventing member 16 for preventing the transmission of collection fluid may also abut the back surface of the membrane filter 8 during recovery, as shown in Fig. 7.

In the present embodiment, a spiral flow path formed by the spacer 121 of the collection fixture 12 is used as a flow path capable of selectively supplying an approximately uniform optimum shear force, however, the present invention is not limited to this configuration. The flow path may also be configured as one or a plurality of straight lines, one or a plurality of curves, or ma combination of more than one straight line and more than one curve. For example, the flow path may be a linear flow path that transects the membrane filter, and a spiral flow path extending from the center to the margin of the membrane filter, or from the margin to the center of the membrane filter. The flow path preferably has an essentially uniform cross section on the membrane filter. Since the shear force of the collection fluid that acts on the cells is rendered nearly uniform in the flow path lengthwise direction by having a substantially constant flow path cross section surface area, the near-uniform shear flow of the entire length of the flow path, that is, across the entire surface of the membrane filter, can be optionally adjusted just by selecting the flow volume of the collection fluid supplied to the flow path. Therefore, damage to cells is prevented to the lowest limit possible, that is, to the lowest limit necessary for the recovery of cells on the membrane filter, an a near-uniform shear force can be readily adjusted and supplied.

Although the collection fixture 12 is provided with a reservoir 125 for recovering free cells in the present embodiment, the reservoir need not necessarily be provided inasmuch as the freed cells may be recovered by detaching the collection fixture 12 from the membrane filter 8 to collect the cells suspended in the suspension fluid on the membrane filter 8.

The cells may be optionally processed while on the membrane, for example, during the reaction time using an optional reagent at a specific reaction temperature. The reagent for processing the cells may be any optional cell processing reagent, for example, staining reagent for staining specific cells, and staining accelerator. Fluorescence-labeled antibody, fluorescent nuclear colorant and the like are desirable staining reagents. For example, cancer cells among the cells to be processed on the membrane filter may be fluorescence labeled using a specific fluorescence-labeled antibody to mark the specific cancer cells. Recovered cells can be supplied to a flow cytometer to detect the presence of cancer cells by detecting fluorescence.

The recovered cells may also be examined microscopically or by flow cytometry. Morphological anomalies are detectable because the recovered cells substantially retain morphological information in the method of the present embodiment, and thus may be used for diagnosing cancer and the like.

Although cells on the membrane filter are collected in an optional liquid using a shear force in the present embodiment, the collection object need not be limited to cells inasmuch as, for example, carrier particles and the like sensitized to antigens or antibodies may be collected.

### (Second Embodiment)

The embodiment of the cell processing apparatus of the present invention is described in detail below with reference to the accompanying drawings.

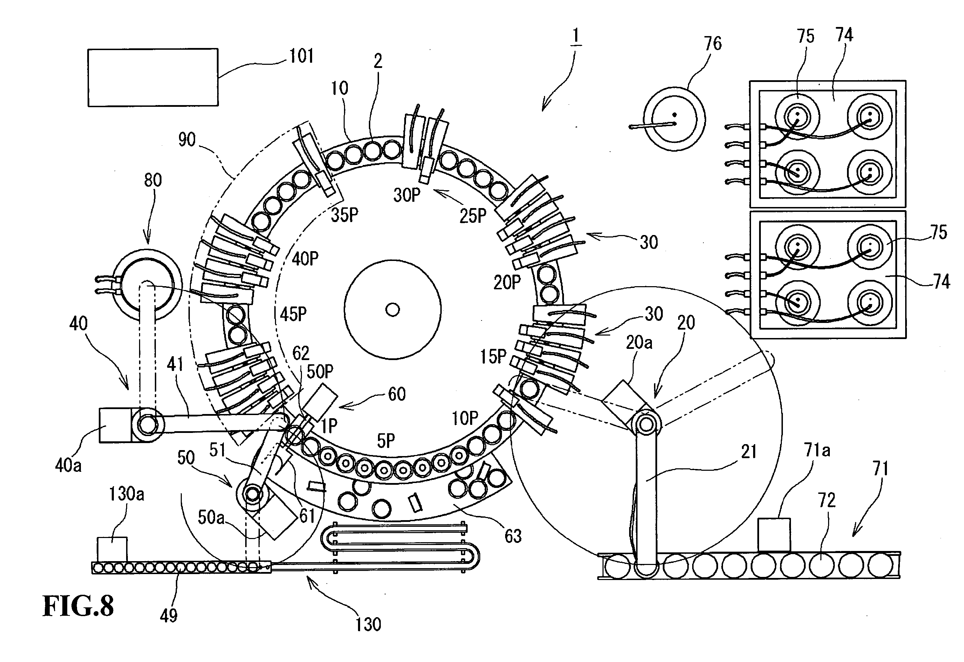

Fig. 8 is a plan view of the cell processing apparatus of an embodiment of the present invention. The cell processing apparatus 1 filters cells in a cell suspension fluid using a membrane filter, subjects the filtered cells to processing such as a reagent process such as staining the cells and washing process using a washing solution on the membrane filter, and subsequently recovering the cells on the membrane filter in a collection fluid.

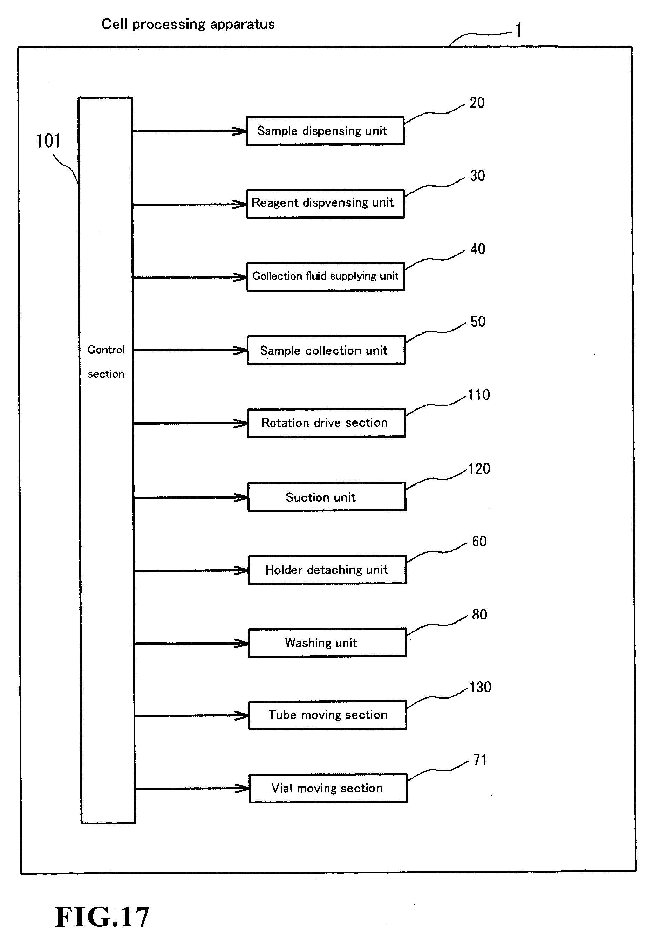

As shown in Figs. 8 and 17, the cell processing apparatus 1 is mainly configured by a holder setting section 10 for deploying a plurality of holders 2 provided with a filter (membrane filter), sample dispensing unit 20 as a first dispensing section for dispensing cell suspension fluid containing cells to the holder 2, reagent dispensing unit 30 as a second dispensing section for dispensing reagent to the holder 2, collection fluid supplying unit 40 as a collection fluid supplying section for supplying collection fluid to separate the reagent-treated cells on the membrane filter, sample collection unit 50 for recovering the samples in the collection fluid, holder detaching unit 60 for removing the used holder 2 from the holder setting section 10, washing unit 80 for washing the collection fluid supplying unit 40, vial moving section 71 for moving vials containing cell suspension fluid, tube moving section 130 for moving tubes containing samples recovered from the collection fluid by the collection unit 40, rotation drive section 110 for rotating the holder setting section, suction unit 120 for suctioning liquid on the filter of the holder 2, and control section 101. The control section 101 is electrically connected to the sample dispensing unit 20, reagent dispensing unit 40, sample collection unit 50, rotation drive section 110, suction unit 120, holder detaching unit 60, washing unit 80, tube moving section 130, and via moving section 71.

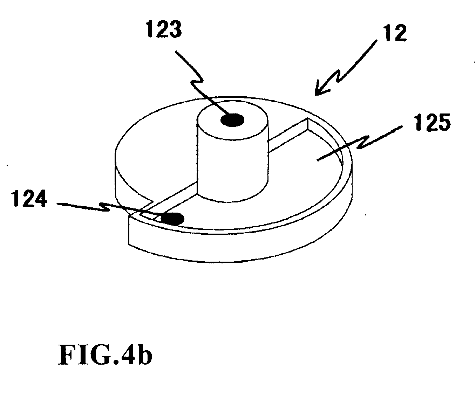

The holder setting section 10 is ring shaped with a plurality of concavities 11 (refer to Figs. 3 and 4) formed at predetermined intervals along the circumference. a detachable holder 2 provided with a filter is disposed within each concavity 11. Thus, the holders 2 are disposed at predetermined intervals in a circular arrangement. The holder setting section 10 is rotatable by a drive means (rotation drive section 110) not shown in the drawings, such that this drive means is capable of moving each holder 2 to positions for various processing such as a sample dispensing position and reagent dispensing position and the like. The holder setting section 10 can be rotated to readily realize automatic processing of a plurality of processing objects by rotating the holder setting section 10 with the circularly deployed holder 2.

The holder 2 is configured as a short cylinder made of synthetic resin such as polypropylene or the like. Approximately 10 percent by weight glass fibers may be mixed with the synthetic resin as reinforcement. A filter 3 for capturing cells in a cell suspension fluid is installed near the endface 2a (the endface on the side inserted in the concavity 11.) of the holder 2. A ring lock 4 having an L-shaped cross section is formed on the outer circumferential surface of the other endface of the holder 2. The ring lock 4 is configured by a flange 4a that projects from the outer surface of the holder 2 in the radial direction, and a perpendicular part 4b that extends from the endface on one side from the leading edge of the flange 4a in the axial direction. A first hook 4c projecting inward in the radial direction is formed at the leading edge of the perpendicular part 4b. The first hook 4c functions to detachably lock the holder 2 to the holder setting section 10 by engaging a second hook 5b projecting outward in the radial direction and formed on the outer circumference of a rink lock 5 rising at the opening margin of the concavity 11 of the holder setting section 10.

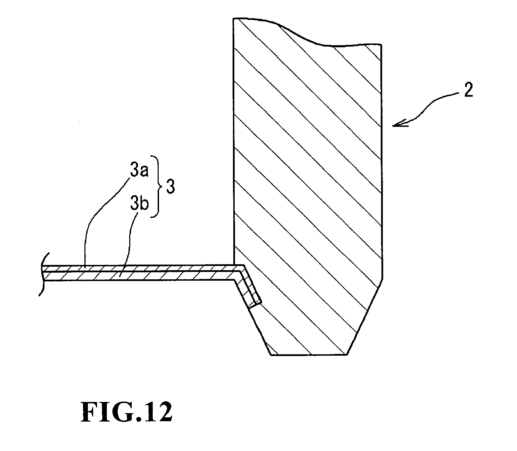

As shown in Fig. 12, the filter 3 has a dual layer structure including a membrane filter 3a for capturing cells, and a mesh filter 3b for supporting the membrane filter 3a. Since a direct reaction of cells in contact with reagent is initiated, the membrane filter 3a is preferably made of a material to which the cells do not readily adhere and has suitable chemical resistance, for example, hydrophilic polytetrafluoroethylene, hydrophilic nylon, hydrophilic polyvinylidene fluoride, hydrophilic polypropylene, hydrophilic polysulfone, hydrophilic polyether sulfone and the like. The pore diameter is selected in accordance with the size of the cells to be processed, and although not specifically limited in the present invention, a pore diameter of 1 to 5 µm is normally used. The mesh filter 3b is provided to prevent deformation of the membrane filter 3a when liquid is dispensed and the like, and its mesh-like structure is formed of synthetic resin such as nylon or the like which is stronger and less flexible than the membrane filter 3a. The mesh filter is not specifically limited, although a pore diameter of 200 to 500 µm is usual. The dual layer structure of the filter 3 can minimize deformation, prevent damage to the filter 3 during cell processing and cell recovery operations. Thus, the function of the filter 3 can be adequately realized.

The filter 3 is arranged so that the membrane filter 3 a and mesh filter 3b overlay a metal die, and is integratedly formed with the holder 2 by an outsert press using the synthetic resin that configures the holder 2. Thus, a holder 2 with attached filter 3 can be manufactured using a few processes. Moreover, the filter 3 and holder 2 can be manufactured separately, and thereafter the filter 3 can be adhered to the holder 2 using adhesive or the like.

The sample dispensing unit 20 is provided with an arm 21 which is vertically movable on the Z-axis and rotatable on the Z-axis (perpendicular axis), and a sample dispensing pipette mounted on the opposite side of the leading end of the rotating arm 21. The arm 21 is configured so as to be vertically movable and rotatable via a stepper motor 20a. Furthermore, the sample dispensing unit 20 is provided with a pumping means not shown in the drawing for suctioning and dispensing sample via the sample dispensing pipette. Suspension fluid containing cells and accommodated in the vial 72 is suctioned by the sample dispensing pipette from the vial 72 transported by a vial moving section 71 which is a belt conveyor, and the cell suspension fluid is dispensed onto the filter 3 in the holder 2. The vial moving section 71 is provided with a drive motor 71a for moving the belt conveyor.

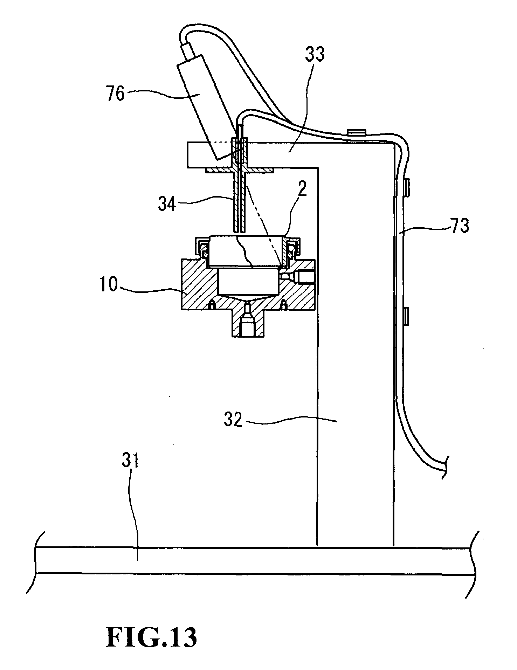

As shown in Fig. 13, the reagent dispensing unit 30 is mainly configured by a column 32 rising from a base plate 31 disposed on the cell processing apparatus 1, arm 33 projecting horizontally from the top end of the column 32, and reagent dispensing pipette 34 held in a perpendicular orientation by the arm 33. A reagent injecting tube 73 is connected to the base end pf the reagent dispensing pipette 34, and the other end of the reagent injecting pipette 34 is connected to a reagent bottle 75 disposed in a reagent container section 74 (refer to Fig. 8) through a pumping means not shown in the drawing.

The reagent dispensing unit 30 is disposed along the outer surface of the holder setting section 10 for dispensing reagent to a plurality of holders arranged in the holder setting section 10. When rotated to the reagent dispensing position, the pumping means is actuated and a predetermined amount of reagent is dispensed onto the filter 3 held by the holder 2 from the tip of the reagent dispensing pipette 34.

A liquid level sensor 76 is mounted on the arm 33 to detect a liquid on the filter 3, and is configured so as to detect the distance to the liquid surface when detecting the level of the liquid on the filter 3. The actuation of the suction means, which produces a negative pressure on the back side of the filter 3 (the opposite side form the side of the cell suspension fluid where the reagent is dispensed) of the holder 2 can be stopped in accordance with the signal from the liquid level sensor 76 as described later.

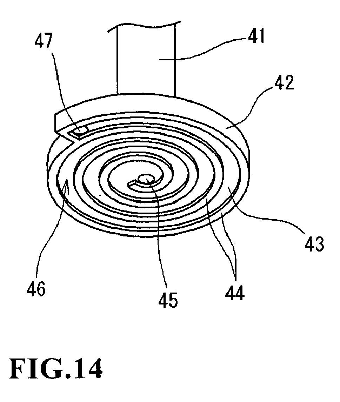

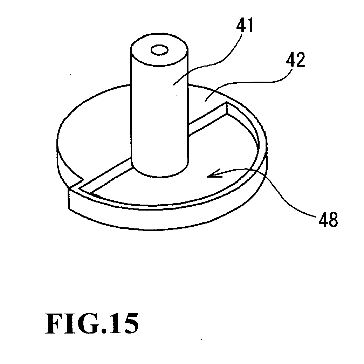

The collection fluid supplying unit 40 is provided with an arm 41 which is vertically movable on the Z-axis and rotatable about the Z-axis similar to the sample dispensing unit 20. The arm 41 is configured so as to be vertically movable and rotatable via a stepper motor 20a. A cylinder 41 for collecting reagent-treated cells on the filter 3 (refer to Figs. 14 and 15) is connected near the tip of the arm 41. A collection fluid supplying tube (not shown in the drawings) is connected to the base side of the cylinder 41, and collection fluid held in a container can be supplied to the filter 3 through the collection fluid supplying tube and cylinder 41 by a pumping means also not shown in the drawings.

As shown in Figs. 14 and 15, the tip of the cylinder 41 is provided with a collection fluid supplying member 42 for supplying collection fluid in a uniform flow to the surface of the filter 3. The collection fluid supplying member 42 is provided with an opposing surface 43 disposed facing the cell existence region of the filter 3, a spacer 44 arranged on the opposing surface 43 to regulate the spacing between the opposing surface 43 and the filter 3, and a supply port 45 for supplying collection fluid.

In the collection fluid supplying member 42, collection fluid is supplied from the supply port 45 to the flow path 46 formed by the spacer 44 and opposing surface 43 to the cell existence region on the filter 3. In this case, the collection fluid supplying member 42 is disposed with the spacer 44 in contact with the cell existence region of the filter 3 in the process in which collection fluid is supplied to release the cells. The collection fluid is caused to flow parallel to the surface of the filter 3 by supplying the collection fluid to a flow path that is parallel to the filter 3 and the cells on the filter 3 by arranging the collection fluid supplying member 42 so that the spacer 44 is in contact with the cell existence region of the filter 3. Thus, collection fluid can be supplied by causing a uniformly strong shearing force to act on the cell existence region on the surface of the filter, thereby releasing the cells from the membrane filter without damaging the morphology of the cells, and allowing the efficient recovery of the cells dispersed in the collection fluid. Moreover, since the flow path is spiral, a uniformly strong flow of collection fluid can be supplied to the cell existence region on the surface of the circular filter surface. The shear force that release the cells from the filter 3 can be adjusted by the flow volume per unit time of the supplied collection fluid, height of the spacer 44, and the width of the flow path formed by the spacer 44, and thus can be suitably set in accordance with the amount and condition of the cells to be processed.

After the collection fluid supplied to the flow path has flowed to the spiral cell existence region, the collection fluid is discharged to a reservoir 48, which stores the collection fluid, via a discharge port 47 for discharging collection fluid that contains cells separated from the filter 3, the reservoir 48 being provided near the end of the spiral flow path. The reservoir 48 is formed as a concavity on the opposite side from the opposing surface 43 in the collection fluid supplying member 42.

The sample collecting unit 50 is provided with an arm 51 that is vertically movable on the Z-axis and rotatable on the Z-axis, and a sample collecting pipette (not shown in the drawing) mounted at the leading end (the end on the opposite side from the rotating shaft) of the rotating arm 51, similar to the sample dispensing unit 20. The arm 51 is configured so as to be vertically movable and rotatable via a stepper motor 50a. Furthermore, the sample collecting unit 50 is provided with a pumping means not shown in the drawing for suctioning and dispensing sample via the sample collecting pipette. The sample collecting unit 50 recovers the collecting fluid (including cells released from the filter 3) within the reservoir 48 of the collection fluid supplying member 42. The suction port of the sample collecting pipette is disposed so as to be offset approximately 3 to 20 degrees from the axial center of the sample collecting pipette, and the suction port can be positioned in the collection fluid within the reservoir 48 by vertically moving and rotating the arm 51 so as to suction the collection fluid when a pump means not shown in the drawing is actuated.

After the collection fluid has been suctioned, the arm 51 is rotated a predetermined angle, and the sample collecting pipette discharges the collection fluid into the tube 49 which is moved by the tube moving means 130 configured by a belt conveyor. The tube moving section 130 is provided with a drive motor 130a for moving the belt conveyor.

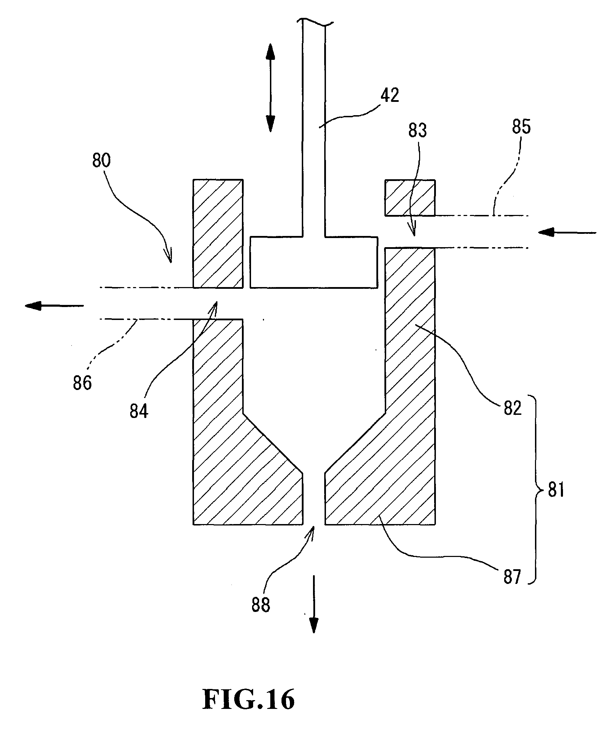

The collection fluid supplying member 42 of the collection fluid suctioned from the reservoir 48 is washed by the washing unit 80. As shown in Fig. 16, the washing unit 80 is configured by a cylinder 81 with a bottom and open top, and a washing solution inlet 823 and outlet 84 are formed in the side wall 82 of the cylinder 81. A tube 85 for supplying washing solution and tube 86 for discharging washing solution are respectively connected to the inlet 83 and outlet 84. The inlet 83 and outlet 84 are connected to a pumping means not shown in the drawing. The washing solution is supplied from a washing solution bottle 76 into the washing unit 80 through the inlet 83 via the pumping means. The washing solution supplied into the washing unit 80 is suctioned and discharged through the outlet 84 by the pumping means. Furthermore, a discharge port 88 is formed in the bottom 87 of the cylinder 81, and the washing solution in the cylinder 81 is suctioned by a suction means not shown in the drawing. While the collection fluid supplying member 42 moves vertically a plurality of times in the cylinder 81 the collection solution supplying member 42 is washed by the washing solution supplied from the inlet 83.

The holder detaching unit 60 has an arm 62 at the leading end of which is provided grips 61 that can open and close, and the arm 62 is capable of moving vertically and advancing and retracting. From the retracted position, the arm 62 advances and the grips 61 close to grip the open part of the holder 2, and in this condition the arm 62 is raised and removes the holder 2 from within the concavity 11. The arm 62 is advanced, the grips 61 open and the hold is released to drop the holder 2 into a holder disposal section 63. The used holders 2 accumulated in the holder disposal section 63 are collected by the user and discarded.

In Fig. 8 shows a light shield cover 90 that is provided to prevent light from causing fading of the fluorescent material used to stain the cells when the laser light irradiates the flow cytometer to excite the fluorescent material staining the cells.

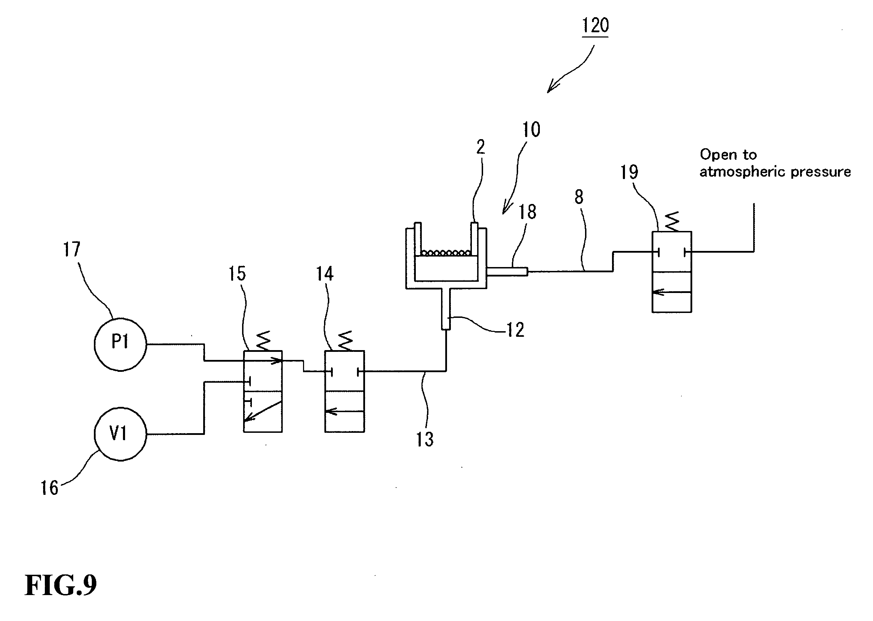

The structure of the suction unit 120, device for suctioning the liquid on the filter 3, and device for exerting pressure on the back side of the filter 3 are described below with reference to Fig. 9.

The suction unit 120 is configured by a holder setting section 10, first valve 14, second valve 15, third valve 19, negative pressure source 16, and positive pressure source 17. One end of a pipe 13 is connected to the first port 12 formed on the reverse side of the holder 2, and the other end of the pipe 13 is connected to the positive pressure source 17, and negative pressure source 16 forming the suction unit, through the first valve 14 and second valve 15. One end of an air tube 8 is connected to the second port 18 formed on the side surface of the holder setting section 10, and the other end of the air tube 8 is open to the atmosphere through the third valve 19. The first valve 14 and third valve 19 are valves whose function is to open and close, whereas the second valve is a switch valve for switching the flow path.

The first valve 14 is open and the third valve 19 is closed when the liquid dispensed to the filter 3 is suctioned through the filter 3 so as to leave some portion of the liquid remaining on the filter 3. Then, after the second valve 15 has been switched to the negative pressure source side, the negative pressure source is actuated to exert a negative pressure on the back side of the filter 3. Thus, the liquid on the filter 3 can be suctioned through the filter 3. The distance to the fluid surface of the liquid on the filter 3 is detected by the liquid level sensor 76, and the first valve 14 is closed and third valve 19 is opened in accordance with the signal from the liquid level sensor 76 to restore atmospheric pressure on the back side of the filter 3. Therefore, a predetermined amount of liquid is assured of remaining on the filter 3, and unnecessary liquid is suctioned.

When the cells subjected to predetermined processing such as reagent processing and washing are recovered in the supplied collection fluid, the third valve 19 and first valve 14 are opened and the second valve is switched to the negative pressure side, and after the liquid in the holder setting section 10 and tube moving section 13 is discharged and replaced with air, the third the third valve 19 is closed and the second valve is switched to the positive pressure source 17, then the positive pressure source 17 is actuated and a positive pressure is exerted on the back side of the filter 3. When the collection fluid is supplied to the filter 3 to release the cells on the filter 3, the collection fluid supplied to the filter 3 can not effectively produce a flow parallel to the filter surface because of the leakage that passes through the filter 3 due to the fluid supplying pressure. It is believed that the filter 3 flexes so as to project downward, and this causes turbulence in the collection fluid flow that prevents cells from being efficiently recovered. However, flow leakage and deformation of the filter 3 can be prevented by using air to exert a positive pressure on the back side of the filter 3. As a result, the collection fluid can be efficiently and smoothly supplied to the cell existence region of the filter 3, and cells on the filter 3 can be efficiently collected.

It is desirable that the first valve 14 is closed after a predetermined positive pressure has been exerted on the back side of the filter 3. After a predetermined pressure has been exerted on the back side of the filter 3, the space on the back side of the filter 3 can be rendered as a sealed space by blocking the communicating path between the back side of the filter 3 and the positive pressure source 17, such that there is minimal deformation of the filter 3 when collection fluid is supplied to the filter 3.

The predetermined pressure (positive pressure) is preferably set so as to be greater than the hydraulic pressure of the collection fluid supplied to the filter 3, and less than the bubble point of the filter 3 so that air does not pass through the filter 3. When collection fluid is supplied to the filter 3 under these conditions, flow leakage and deformation of the filter 3 is effectively prevented and the collection fluid flow is efficiently formed.

The rotation drive section 110 shown in Fig. 17 is described below. The rotation drive section 110 is provided with a rotation drive source (not shown in the drawing) for rotating the holder setting section 10 at a predetermined pitch. The holder setting section 10 rotates and stops in accordance with number of pulses of the drive pulse signals supplied to the rotation drive section 110 by the control section 10.

The control section 101 determines the amount of rotational movement of the holder setting section 10 from the origin position by counting the number of pulses in the supplied drive pulse signals so as to control the rotation of the holder setting section 10. The control section 101 controls the rotation drive section 110 so as to rotate the holder setting section 10 counterclockwise one pitch per 3 minutes.

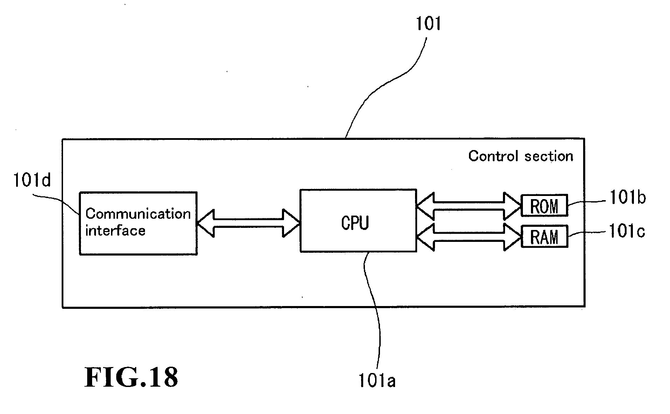

As shown in Fig. 18, the control section 110 is mainly configured by a CPU 101a, ROM 101b, RAM 101c, and communication interface 101d.

The CPU 101a executes computer programs stored in the ROM 101b, and computer programs read from the RAM 101c. The ROM 101b stores computer programs executed by the CPU 101a, and data used by these computer programs. The RAM 101c is used to read computer programs stored in the ROM 101b. The RAM 101c is also used as a work area for the CPU 101a when executing these computer programs.

The communication interface 101d functions to send commands from the CPU 501a for driving the various sections including the sample dispensing unit 20, reagent dispensing unit 30, collection fluid supplying unit 40, sample collection unit, rotation drive unit 110, suction unit 120, holder detaching unit 60, washing unit 80, tube moving section 130, and vial moving section 71.

An example of the cell processing method used by the cell processing apparatus of the present embodiment is described below. In the method of the present embodiment, the cells in the cell suspension fluid are cells collected from a patient, and information aiding diagnosis is obtained through examination of these cells. In diagnosing cervical cancer, for example, a cell suspension fluid is used in which cells originating in the uterus are suspended in a solvent.

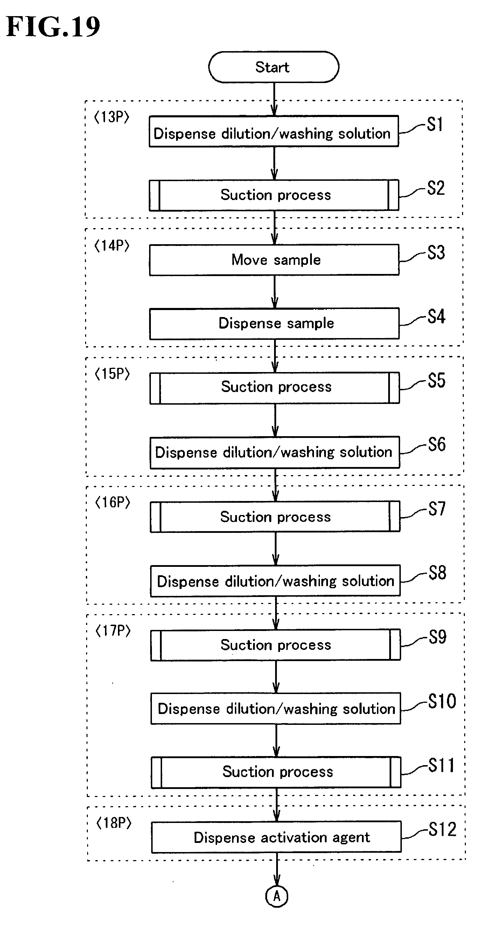

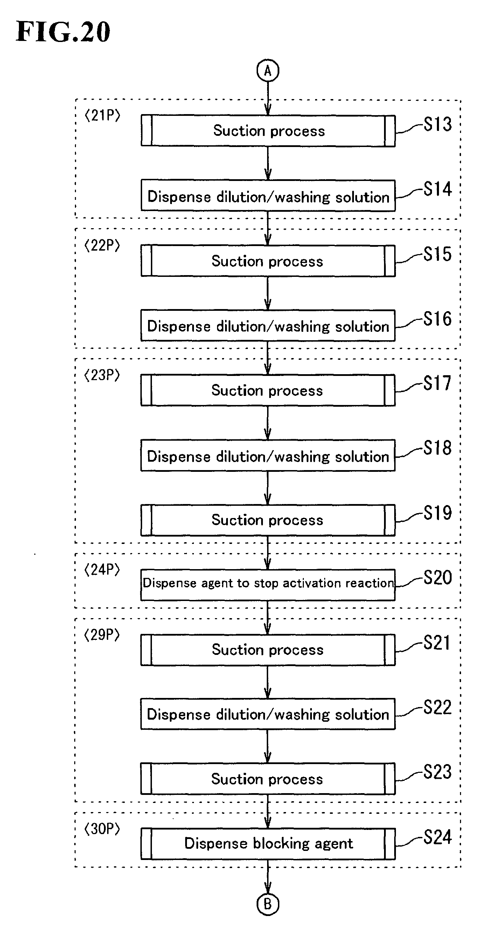

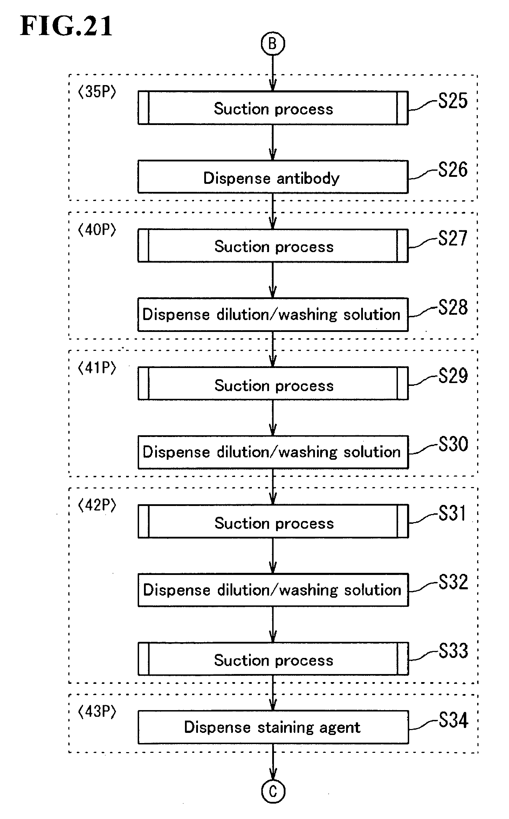

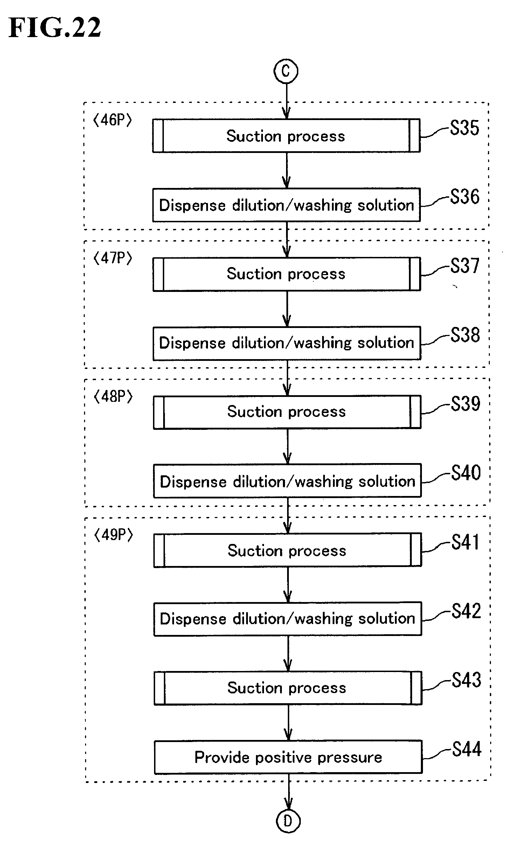

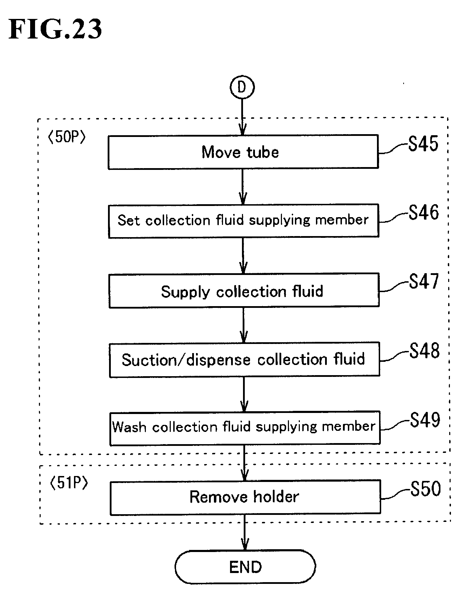

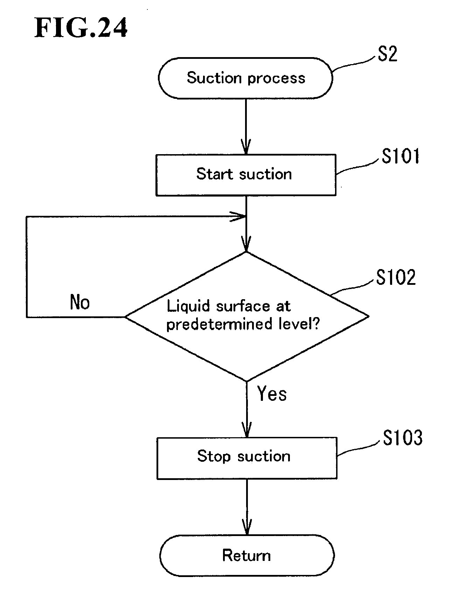

As shown in Fig. 8, the holder setting section 10 can accept fifty-one individual holders 2, and is rotated by the rotation drive section 110 counterclockwise one pitch per 3 minutes to perform processes at predetermined positions. That is, processes are performed at fifty-one predetermined positions. Position number 1P through 51P are respectively appended to the fifty-one predetermined positions. The processes involving the holder 2 accommodated by the holder setting section 10 are described via the position numbers with reference to the flow charts shown in Figs. 19 through 23 and 24. The control of the rotation drive section 110 by the control section 101 is omitted from this description since the holder setting section 10 is only rotated counterclockwise one pitch per 3 minutes.

### <Positions 1P through 12P>

The user first installs holders 2 at positions 1P through 12P of the holder setting section 10.

### <Position 13P>

Then, the control section 101 dispenses dilution/washing solution onto the filter 3 of the holder 2 at position 13P by controlling the reagent dispensing unit 30 (step S1). Next, the control section 101 performs a suction process by controlling the suction unit 120 (step S2).

The suction process of step S2 is describe din detail below with reference to the flow chart of Fig. 17. The control section 101 controls the suction unit 120 to start to suction liquid on the filter 3 to create a weak negative pressure from the back side of the filter 3 (step S101). The liquid level on the filter 3 is detected by the liquid level sensor 76, and a determination is made as to whether or not this is a predetermined level (step S102). When the level is not a predetermined level, step S102 is repeated until the predetermined liquid level is detected. when the predetermined liquid level is detected, the control section 101 opens the first valve 14 and closes the third valve 19 to open the back side of the filter 3 to atmospheric pressure, and immediately thereafter the third valve 19 is closed and the suctioning by negative pressure is stopped (step S103) . Thus, the filter 3 is normally wetted with the liquid by detecting the liquid level on the filter 3. Subsequent suctioning processes are identical to step S2 and therefore further description is omitted.

### <Position 14P>

The control section 101 moves the vial 72 containing cell suspension fluid (cells approximately 3 to 200 µm in diameter; cell content approximately 1×10⁵ in 1 mL fixing solution) prepared from cervical scraping specimen to the suction position by the vial moving section 71 (step S3), and suctions the cell suspension fluid from the transported vial 72 via the sample dispensing pipette, and dispenses the suspension onto the filter 3 of the holder 2 (step S4). The filter 3 has a dual layer structure in which a hydrophilic PTFE flat membrane filter (pore diameter: 0.4 to 5 µm) is laminated over a reticulate mesh sheet approximately 60 µm.

### <Positions 15P and 16P>

At position 15P, the control section 101 performs a suction process by controlling the suction unit 120 (step S5). Then, the control section 101 dispenses dilution/washing solution onto the filter 3 of the holder 2 by controlling the reagent dispensing unit 30 (step S6).

At position 16P, the control section 101 performs a similar process (steps 7 and 8).

### <Position 17P>

At position 17P, the control section 101 performs a suction process by controlling the suction unit 120 (step S9). Then, the control section 101 dispenses dilution/washing solution onto the filter 3 of the holder 2 by controlling the reagent dispensing unit 30 (step S10). Next, the control section 101 performs a suction process by controlling the suction unit 120 (step S11).

### <Position 18P>

At position 18P, the control section 101 dispenses an activating agent from the reagent bottle 75 by controlling the reagent dispensing unit 30 (step S12).

### <Positions 19P and 20P>

At positions 19P and 20P, the control section 101 does not perform a process (this is the cell and activating agent reaction time).

### <Positions 21P and 22P>

At position 21P, the control section 101 performs a suction process by controlling the suction unit 120 (step S13). Then, the control section 101 dispenses dilution/washing solution onto the filter 3 of the holder 2 by controlling the reagent dispensing unit 30 (step S14). At position 22P, the control section 101 performs a similar process (steps 15 and 16).

### <Position 23P>

At position 23P, the control section 101 performs a suction process by controlling the suction unit 20 (step S17). Then, the control section 101 dispenses dilution/washing solution from the reagent bottle 75 onto the filter 3 of the holder 2 by controlling the reagent dispensing unit 30 (step S18). Next, the control section 101 performs a suction process by controlling the suction unit 120 (step S19).

### <Position 24P>

At position 24P, the control section 101 dispenses an activation reaction damping agent from the reagent bottle 75 by controlling the reagent dispensing unit 30 (step S20).

### <Positions 25P through 28P>

At positions 25P through 28P, the control section 101 does not perform a process (this is the activation reaction damping agent and activation agent reaction time).

### <Position 29P>

At position 29P, the control section 101 performs a suction process by controlling the suction unit 120 (step S21). Then, the control section 101 dispenses dilution/washing solution from the reagent bottle 75 onto the filter 3 of the holder 2 by controlling the reagent dispensing unit 30 (step S22) . Next, the control section 101 performs a suction process by controlling the suction unit 120 (step S23).

### <Position 30P>

At position 30P, the control section 101 dispenses blocking agent from the reagent bottle 75 onto the filter 3 by controlling the reagent dispensing unit 30 (step S24).

### <Positions 31P through 34P>

At positions 31P through 34P, the control section 101 does not perform a process (this is the cell and blocking agent reaction time).

### <Position 35P>

At position 35P, the control section 101 performs a suction process by controlling the suction unit 120 (step S25). Then, the control section 101 dispenses antibody from the reagent bottle 75 onto the filter 3 by controlling the reagent dispensing unit 30 (step S26).

### <Positions 36P through 39P>

At positions 36P through 39P, the control section 101 does not perform a process (this is the cell and antibody reaction time).

### <Positions 40P and 41P>

At position 40P, the control section 101 performs a suction process by controlling the suction unit 120 (step S27). Then, the control section 101 dispenses dilution/washing solution from the reagent bottle 75 onto the filter 3 of the holder 2 by controlling the reagent dispensing unit 30 (step 528). At position 41P, the control section 101 performs a similar process (steps 29 and 30).

### <Position 42P>

At position 42P, the control section 101 performs a suction process by controlling the suction unit 120 (step S31). Then, the control section 101 dispenses dilution/washing solution from the reagent bottle 75 onto the filter 3 of the holder 2 by controlling the reagent dispensing unit 30 (step S32). Then, the control section 101 performs a suction process by controlling the suction unit 120 (step S33).

### <Position 43P>

At position 43P, the control section 101 dispenses staining solution from the reagent bottle 75 by controlling the reagent dispensing unit 30 (step S34).

### <Positions 44P and 45P>

At positions 44P through 45P, the control section 101 does not perform a process (this is the antibody and staining agent reaction time).

### <Positions 46P through 48P>

At position 46P, the control section 101 performs a suction process by controlling the suction unit 120 (step S35). Then, the control section 101 dispenses dilution/washing solution from the reagent bottle 75 onto the filter 3 of the holder 2 by controlling the reagent dispensing unit 30 (step S36). At positions 47P and 48P, the control section 101 performs a similar process (steps 37 through 40).

### <Position 49P>

At position 49P, the control section 101 performs a suction process by controlling the suction unit 120 (step S41). Then, the control section 101 dispenses dilution/washing solution from the reagent bottle 75 onto the filter 3 of the holder 2 by controlling the reagent dispensing unit 30 (step S42). Next, the control section 101 performs a suction process by controlling the suction unit 120 (step S43).

After the third valve 19 is opened and the first valve 14 is closed and the liquid within the holder setting section 10 and tube moving section 13 is discharged and replaced with air, the third valve 19 is closed and the first valve 14 is opened and a positive pressure is applied to the back side of the filter 3 which is less than the bubble point of the filter 3 and greater than the hydraulic pressure of the collection fluid supplied to the filter 3 during the collection fluid supplying time, and thereafter the first valve 14 is closed and the back side of the filter 3 is blocked (step S44).

### <Position 50P>

At position 50P, the control section 101 moves the tube 49 to the collection position by controlling the tube moving section 130 (step S45).

The arm 41 of the collection fluid supplying unit 40 is rotated and lowered, and the spacer 44 of the collection fluid supplying member 42 is disposed in contact with the cell existence region on the filter 3 as shown in Figs. 7 and 8 (step S46). Collection fluid is supplied from the supply port 45 positioned in the center of the collection fluid supplying member 42 to the spiral flow path 46 formed by the spacer 44 and opposing surface 43 to the cell existence region on the filter 3. Thus, a uniform parallel flow is formed on the surface of the filter 3, and the cells on the filter 3 are released and recovered in the collection fluid by this flow. The release of cells strongly adhered to the filter 3 and dispersion of analogous cells is promoted by the reciprocating vertical movement of the collection fluid supplying member 42.

Then, the control section 101 houses the collection fluid supplying member 42 in the reservoir 48, and the collection fluid which contains the cells released from the filter 3 are recovered and injected into the tube 49 by controlling the sample collection unit 50 (step S48). The control section 101washes the collection fluid supplying member 42 with washing solution by controlling the collection fluid supplying unit 40 and the washing unit 80 (step S49). The sample can be measured and analyzed by setting the tube 40 at a predetermined position in the flow cytometer.

### <Position 51P>

Then the control section 101 removes the holder 2 installed in the holder setting section 10 and disposes of the holder 2 in the holder waste section 63 by controlling the holder detaching unit 60 (step S50).

Although the user installs the holders 2 in the holder setting section 10 in the present embodiment, a supplying section may also be provided to automatically supply the holders 2 to the holder setting section 10.

## Claims

1. A cell processing method for processing and collecting cells in a cell suspension fluid comprising the steps of:
filtering a cell suspension fluid in which cells are suspended using a membrane filter to capture the cells on the membrane filter;
processing the cells on the membrane filter with reagent;
supplying a collection fluid to the membrane filter to produce a shearing force that acts on the cells processed with reagent and transfer the cells from the membrane filter into the collection fluid; and
collecting the collection fluid in which the cells transferred from the membrane filter are suspended.

2. The cell processing method of claim 1, wherein the reagent is a staining reagent for staining specific cells.

3. The cell processing method of claim 1 or 2 further comprising a step for washing the cells on the membrane filter using a washing liquid.

4. The cell processing method of any one of claim 1 to 3,
wherein a collection fluid supplying member is disposed in a cell existence region of the membrane filter in which the captured cells are present, wherein the collection fluid supplying member comprises an opposing surface to be disposed opposite the cell existence region, a spacer for regulating a clearance between the membrane filter and the opposing surface and a supply port for supplying the collection fluid, wherein the collection fluid is supplied from the supply port.

5. The cell processing method of claim 4, wherein the collection fluid produces a shearing force on the cells in the cell existence region when the collection fluid is supplied through a flow path formed by the spacer and the opposing surface.

6. The cell processing method of claim 5, wherein the flow path is formed in a spiral shape.

7. The cell processing method of any one of claim 1 to 6, wherein filtering is performed so that a solvent of the cell suspension fluid remains on the membrane filter.

8. The cell processing method of any one of claim 1 to 7, wherein the transfer of the cells from the membrane filter into the collection fluid is accomplished under a condition that the collection fluid does not pass through the membrane filter.

9. The cell processing method of claim 8, wherein the condition that the collection fluid does not pass through the membrane filter is accomplished by supplying air to a first surface of the membrane filter which does not contact with the collection liquid substantially, at a pressure below a bubble point at which a bubble is generated from the membrane filter and above a fluid pressure on a second surface of the membrane filter which contacts with the collection liquid.

10. The cell processing method of any one of claim 1 to 9, wherein the membrane filter is formed of a hydrophilic polymer material.

11. The cell processing method of claim 10, wherein the hydrophilic polymer material is selected from a group consisting of hydrophilic polytetrafloroethylene, hydrophilic nylon, hydrophilic polyvinylidene fluoride, hydrophilic polycarbonate, hydrophilic polypropylene, hydrophilic polysulfone, and hydrophilic polyether sulfone.

12. A cell processing apparatus for processing cells using a membrane filter comprising:
a setting section in which a holder having a membrane filter is set;
a first dispensing section for dispensing a cell suspension fluid that contains cells to the holder set in the setting section;
a second dispensing section for dispensing a reagent to the holder set in the setting section;
a liquid supplying section for supplying a separating liquid to separate the cells from the membrane filter in the holder arranged in the setting section; and
a control section for controlling the first dispensing section to dispense the cell suspension fluid to the holder, the second dispensing section to dispense the reagent to the holder to which the cell suspension fluid has been dispensed, and the liquid supplying section to supply the separating liquid to separate the cells treated with the reagent from the membrane filter in the holder to which the reagent has been dispensed.

13. The cell processing apparatus of claim 12 further comprising an aspirating section for aspirating through the membrane filter a liquid that has been dispensed to the holder arranged in the setting section.

14. The cell processing apparatus of claim 13, wherein the control section controls the aspiration operation of the aspirating section so that a solvent of the cell suspension fluid remains on the membrane filter.

15. The cell processing apparatus of claim 14 further comprising a liquid level sensor for detecting a liquid on the membrane filter.

16. The cell processing apparatus of any one of claim 12 to 15, wherein the liquid supplying member comprises an opposing surface disposed opposite a cell existence region on the membrane filter in which the captured cells are present, a spacer for regulating a clearance between the membrane filter and the opposing surface, and a supply port for supplying a liquid.

17. The cell processing apparatus of Claim 16, wherein a flow path is formed in a spiral shape by the cell existence region, opposing surface, and spacer.

18. The cell processing apparatus of any one of claim 12 to 17 further comprising an air pressure source for supplying air pressure to a first surface of the membrane filter which does not contact with the collection liquid substantially.

19. The cell processing apparatus of any one of claim 12 to 18, wherein the setting section is capable of being set the holder in a circular shape, and is configured to be rotatable.
